# EUROPÄISCHE PATENTSCHRIFT

(11) **EP 0 428 479 B1**
(45) Veröffentlichungstag und Bekanntmachung des Hinweises auf die Patenterteilung: **23.11.1994**
(21) Anmeldenummer: 90810834.3
(22) Anmeldetag: 31.10.1990
(51) Int. Cl.: A61F 2/06

(54) **Stent und Katheter zum Einführen des Stents**
Stent and catheter for inserting the stent
Dilatateur et cathéter pour l'introduction du dilatateur

(30) Priorität: 01.11.1989 CH 3946/89
(43) Veröffentlichungstag der Anmeldung: 22.05.1991
(73) Patentinhaber: SCHNEIDER (EUROPE) AG, 8180 Bülach (CH)
(72) Erfinder: Beck, Andreas, W-7746 Hornberg (DE); Nanko, Norbert Anton, W-7800 Freiburg i.Br. (DE)
(74) Vertreter: Groner, Manfred

(56) Entgegenhaltungen:
- WO-A-90/01969
- US-A- 4 636 195
- US-A- 4 739 762

## Beschreibung

### Fassung für folgende Vertragsstaaten : AT, BE, CH, DE, FR, GB, IT, LI, LU, NL, SE

Die Erfindung betrifft einen Stent nach dem Oberbegriff des unabhängigen Patentanspruchs 1 sowie einen Ballonkatheter zum Einführen des Stents nach dem Oberbegriff des Anspruchs 7. Solche Stents bzw. Ballonkatheter sind z.B. aus der US-A-4 739 762 bzw. der US-A-4 636 195 bekannt. Stents und insbesondere intervaskuläre Stents für Angioplastie haben sich in der medizinischen Praxis zur Vermeidung von Okklusionen oder Restenosierungen nach einer transluminalen Angioplastie weitgehend bewährt. Ein bekannter Stent besteht aus einem hülsenförmigen Gitter aus rostfreiem Stahl. Der im Umfang 1,6 oder 3 mm messende Stent wird auf einen gefalteten Ballonkatheter aufgeschoben und Perkutan an die gewünschte Stelle des Blutgefässes gebracht. Durch ein Dilatieren dem Ballonkatheters wird der Stent auf einen Durchmesser von etwa 3 mm aufgeweitet. Der eingesetzte und fixierte Stent wird im Gefäss belassen und in der Regel durch eine sich bildende Neointima überdeckt. Zum Stand der Technik wird auf den Artikel von Julio C. Palmaz in der Zeitschrift Radiology, Juli 1988, 150:1263-1269, hingewiesen.

Die Druckschrift WO-A-9001969 ist nach dem Prioritätstag der Anmeldung veröffentlicht und bildet somit einen Stand der Technik nach Art. 54(3)-EPÜ. Ein Stent mit einer Länge von 2 bis 10 cm, bzw. ein 3-lumiger Ballonkatheter mit zwei Lumen zur Heizung des Balloninnenraums ist aus dieser Druckschrift nicht zu entnehmen.

Der Erfindung liegt die Aufgabe zugrunde, einen Stent der genannten Art zu schaffen, der in seiner Verwendung noch sicherer und einfacher ist und der trotzdem kostengünstiger hergestellt werden kann. Die Aufgabe wird durch die Erfindung gemäss Anspruch 1 gelöst.

Der erfindungsgemässe Stent wird bei Raumtemperatur auf den Ballon des Ballonkatheters aufgeschoben und ist hierbei vergleichsweise fest und steif. Durch leichtes Dilatieren des Ballons kann der Stent auf dem Katheter fixiert werden. Ist der Stent an die gewünschte Stelle, beispielsweise in einen stenosiertem Abschnitt einer Arterie vorgeschoben, so wird er mit einer im Katheter angeordneten Heizeinrichtung erwärmt, bis er radial durch Dilatation des Ballons erweitert werden kann. Der Stent wird soweit erweitert, bis er mit leichtem Druck an der Innenwand des Gefässes anliegt. Die Heizung des Katheters wird nun unterbrochen, wonach der Stent vergleichsweise schnell in den festen Zustand übergeht und hierbei die dilatierte Form beibehält. Im implantierten Zustand ist die Wandstärke des Stent kleiner als im ursprünglichen Zustand und somit etwas flexibler. Im implantierten Zustand kann sich der Stent somit innerhalb gewisser Grenzen an den Verlauf des Gefässes anpassen. Der erfindungsgemässe Stent kann aus einem Kunststoff hergestellt werden, der vermutlich verträglicher ist als Metall. Die Innenseite des Stent kann hierbei vollständig glatt sein, so dass die Gefahr einer Trombose herabgesetzt ist.

Der erfindungsgemässe Stent eignet sich insbesondere für die koronare und peripherale Angioplastie, jedoch sind auch andere Anwendungen denkbar. Beispielsweise könnte der erfindungsgemässe Stent überall dort geeignet sein, wo es gilt, einen Durchgang dauernd oder vorübergehend offen zu halten. Beispiele solcher Durchgänge sind der Gallengang und die Harnröhre.

Der Stent ist beispielsweise durch Extrusion in den unterschiedlichsten Aussen- und Innendurchmessern herstellbar. Somit kann in jedem Fall der Stent mit dem geeigneten Abmessungen zur Verfügung gestellt werden. Für die Behandlung einer Koronararterie wird beispielsweise ein Stent mit kleineren Aussen- und Innendurchmessern gewählt als für die Behandlung einer peripheren Arterie. Ebenfalls wird zur Behandlung eines bogenförmigen Gefässabschnittes in der Regel ein vergleichsweise kurzer Stent verwendet.

Nach einer Weiterbildung der Erfindung ist der Stent aus einem Werkstoff hergestellt, der in Körperflüssigkeit vollständig und biologisch abgebaut wird. Geeignet sind hier insbesondere Werkstoffe aus aliphatischen Polyestern und insbesondere aus Poly (_{E}-caprolacton). Der biologische Abbau dieser Kunststoffe ist bekannt. In der Medizin werden solche Stoffe bereits für die Fixation von Prothesen und als Kapseln für die kontrollierte Abgabe von Medikamenten verwendet. Die Abbaugeschwindigkeit ist beim Werkstoff des erfindungsgemässen Stent etwa so, dass er innerhalb von etwa 2 bis 6 Monaten vollständig oder zum grössten Teil aufgelöst ist. Da der Stent nach vergleichsweise kurzer Zeitdauer an der behandelten Stelle nicht mehr vorhanden ist, ist im Fall der Behandlung einer Stenose die Gefahr einer Trombose kleiner als bei einem dauernd verbleibenden Stent.

Ein Ausführungsbeispiel der Erfindung wird nachfolgend anhand der Zeichnungen näher erläutert. Es zeigen:
Fig. 1 eine perspektivische Ansicht eines erfindungsgemässen Stents,
Fig. 2a und 2b das vordere Ende eines Ballonkatheters mit einem erfindungsgemässen Stent vor und nach der Dilatation, und
Fig. 3a und 3b schematisch ein Schnitt durch ein Gefäss mit eingesetztem Stent vor und nach der Dilatation.

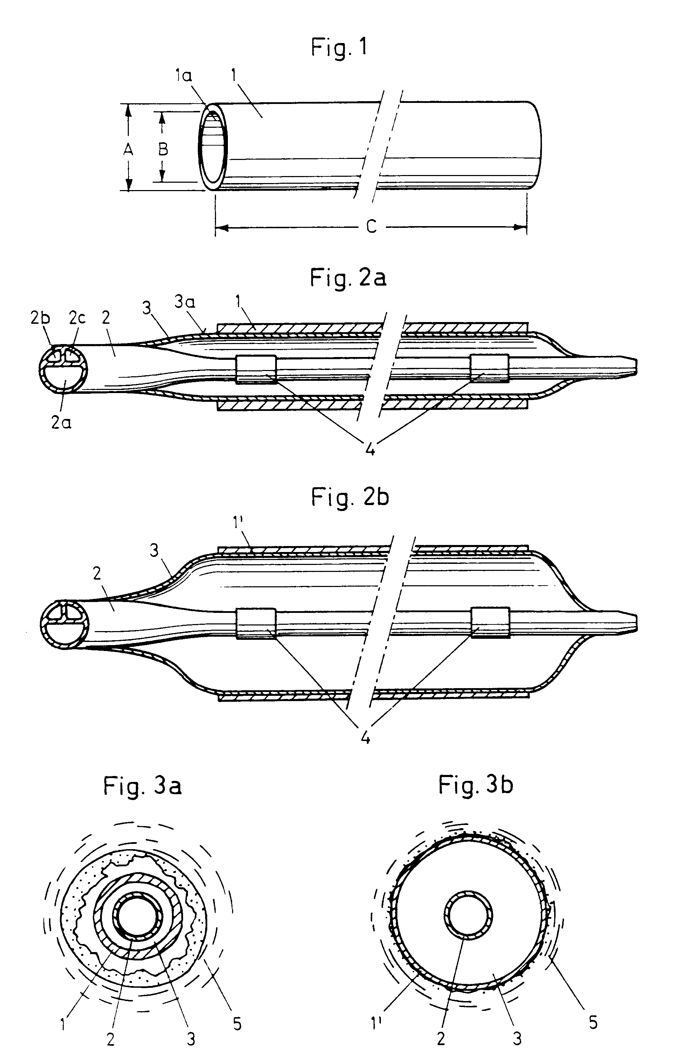

Der in Fig. 1 gezeigte Stent ist ein hohlzylindrischer Körper mit einer Länge C von 2 bis 10 cm. Für die Behandlung einer Koronararterie beträgt der Innendurchmesser B beispielsweise 1,0 ± 0,005 mm und der Aussendurchmesser A 1,6 mm. Zur Behandlung einer peripheren Arterie beträgt der Innendurchmesser B beispielsweise 2,0 mm und der Aussendurchmesser A 3,0 mm Der Stent 1 kann durch Extrusion hergestellt werden. Als Werkstoff eignet sich ein Kunststoff, der im Temperaturbereich von 45 bis 75_{°} Celsius schmilzt oder durch allmähliche Erweichung in den plastischen Zustand übergeht. Als Werkstoff eignen sich aliphatische Poyester und insbesondere Poly (_{E}-caprolacton). Geeignet sind auch Polymere-, die unterhalb einer Temperatur von 45 _{°} Celsius fest sind und die wenigstens oberhalb einer Temperatur von etwa 70 _{°} Celsius in einen nicht kristallinen Zustand übergehen. Geeignete Polymere sind Polycaprolactone, Polyuretane sowie Polyamide.

Von diesen Polymeren sind diejenigen besonders geeignet, die in Körperflüssigkeit biologisch abgebaut werden. Geeignet ist hier insbesondere Poly (_{E}-caprolacton), dessen Abbaubarkeit In Vivo im Journal of Applied Polymer Sciences, Vol. 26, 3779-3787 (1981) beschreiben ist.

Um den Stent 1 an die gewünschte zu behandelnde Stelle zu bringen, wird ein heizbarer Ballonkatheter 3 verwendet. Dieser besitzt einen dreilumigen Schaft 2, wobei ein Lumen 2a zur Durchführung eines Führungsdrahtes dient. In den beiden anderen Lumina 2b und 2c zirkuliert eine Salzlösung, die über hier nicht gezeigte Oeffnungen in den Innenraum des Ballons 3 ein und wieder ausströmt. Die Salzlösung wird mit einer am proximalen Ende des Schaftes 2 angeordneten Heiz- und Pumpeinrichtung erwärmt und gefördert. Es können hier jedoch auch Ballonkatheter verwendet werden, die in an sich bekannter Weise elektrisch, mit Hochfrequenz oder Mikrowellen geheizt werden.

Um den Stent 1 auf dem Ballonkatheter zu befestigen wird dieser vom distalen Ende her auf den gefalteten Ballon aufgeschoben. In vielen Fällen ist der Stent 1 durch die Reibung seiner Innenseite 1 an der Aussenseite 3a des Ballons 3 gegen eine Verschiebung in Längsrichtung genügent fixiert. In den übrigen Fällen wird der Druck im Ballon 3 leicht erhöht. Wie in Fig. 2a gezeigt, wird die Länge des Ballons 3 so gewählt, dass diese etwas grösser ist als die Länge C des Stent 1. Der Stent 1 liegt somit mit seiner ganzen Innenfläche 1 an der Aussenfläche 3a des Ballons an. Bei noch nicht geheiztem Ballonkatheter ist der Stent 1 vergleichsweise fest und steif und wird auch bei einem vergleichsweise hohem Druck im Ballon 3 nicht gedehnt.

Der Katheter wird mit dem aufgesetzten Stent 1 in an sich bekannter Weise eingeführt. Zur Behandlung einer Stenose wird der Katheter mit Hilfe eines hier nicht gezeigten Führungsdrahtes perkutan eingeführt. Die Position des Stent 1 kann mit Hilfe bekannter Markierungsstreifen 4 beispielsweise röntgenographisch verfolgt werden.

Ist der Stent 1 mit dem Ballonkatheter in die gewünschte Stelle gebracht, so wird der Ballon 3 geheizt und der Stent 1 auf eine Temperatur gebracht, in welcher er durch eine Dilatation des Ballones 3 plastisch gedehnt werden kann. Zur Behandlung einer koronaren Arterie wird beispielsweise der Innendurchmesser auf 2,7 und der Aussendurchmesser auf 3,0 mm erhöht. Im Fall einer peripheren Arterie beträgt der Innendurchmesser des dilatierten Stent beispielsweise 5,4 mm und der Aussendurchmesser 6,0 mm. Die wandstärke des dilatierten Stent 1', ist wie aus den Fig. 2a und 2b ersichtlich wesentlich kleiner als beim ursprünglichen Stent 1.

Der Stent 1 wird soweit dilatiert, bis seine Aussenseite mit leichtem Druck an der Innenseite des Gefässes anliegt. Dies ist in den Fig. 3a und 3b schematisch gezeigt. Das Gefäss 5 ist in diesem Fall beispielsweise ein stenosierter Arterienabschnitt 5.

Ist der Stent 1' im Gefäss 5 fixiert, so wird die Heizung des Katheters unterbrochen, worauf die Temperatur des Stent 1' auf die Körpertemperatur sinkt und hierbei wieder den festen Zustand annimmt. Anschliessend wird der Druck im Ballon 3 vermindert und der Katheter in bekannter Weise aus dem Gefäss entfernt.

Ist der Stent 1' aus Poly (_{E}-caprolacton) hergestellt, so wird er in etwa 2 bis 6 Monaten autokatalytisch durch eine hydrolytische Esterspaltung vollständig abgebaut.

### Fassung für folgende Vertragsstaaten : DK, ES, GR

Die Erfindung betrifft einen Stent nach dem Oberbegriff des unabhängigen Patentanspruchs 1 sowie einen Ballonkatheter zum Einführen des Stents nach dem Oberbegriff des Anspruchs 7. Solche Stents bzw. der Ballonkatheter sind z.B. aus der US-A-4 739 762 bzw. der US-A-4 636 195 bekannt. Stents und insbesondere intervaskuläre Stents für Angioplastie haben sich in der medizinischen Praxis zur Vermeidung von Okklusionen oder Restenosierungen nach einer transluminalen Angioplastie weitgehend bewährt. Ein bekannter Stent besteht aus einem hülsenförmigen Gitter aus rostfreiem Stahl. Der im Umfang 1,6 oder 3 mm messende Stent wird auf einen gefalteten Ballonkatheter aufgeschoben und Perkutan an die gewünschte Stelle des Blutgefässes gebracht. Durch ein Dilatieren des Ballonkatheters wird der Stent auf einen Durchmesser von etwa 3 mm aufgeweitet. Der eingesetzte und fixierte Stent wird im Gefäss belassen und in der Regel durch eine sich bildende Neointima überdeckt. Zum Stand der Technik wird auf den Artikel von Julio C. Palmaz in der Zeitschrift Radiology, Juli 1988, 150:1263-1269, hingewiesen.

Der Erfindung liegt die Aufgabe zugrunde, einen Stent der genannten Art zu schaffen, der in seiner Verwendung noch sicherer und einfacher ist und der trotzdem kostengünstiger hergestellt werden kann. Die Aufgabe wird durch die Erfindung gemäss Anspruch 1 gelöst.

Der erfindungsgemässe Stent wird bei Raumtemperatur auf den Ballon des Ballonkatheters aufgeschoben und ist hierbei vergleichsweise fest und steif. Durch leichtes Dilatieren des Ballons kann der Stent auf dem Katheter fixiert werden. Ist der Stent an die gewünschte Stelle, beispielsweise in einen stenosiertem Abschnitt einer Arterie vorgeschoben, so wird er mit einer im Katheter angeordneten Heizeinrichtung erwärmt, bis er radial durch Dilatation des Ballons erweitert werden kann. Der Stent wird soweit erweitert, bis er mit leichtem Druck an der Innenwand des Gefässes anliegt. Die Heizung des Katheters wird nun unterbrochen, wonach der Stent vergleichsweise schnell in den festen Zustand übergeht und hierbei die dilatierte Form beibehält. Im implantierten Zustand ist die Wandstärke des Stent kleiner als im ursprünglichen Zustand und somit etwas flexibler. Im implantierten Zustand kann sich der Stent somit innerhalb gewisser Grenzen an den Verlauf des Gefässes anpassen. Der erfindungsgemässe Stent kann aus einem Kunststoff hergestellt werden, der vermutlich verträglicher ist als Metall. Die Innenseite des Stent kann hierbei vollständig glatt sein, so dass die Gefahr einer Trombose herabgesetzt ist.

Der erfindungsgemässe Stent eignet sich insbesondere für die koronare und peripherale Angioplastie, jedoch sind auch andere Anwendungen denkbar. Beispielsweise könnte der erfindungsgemässe Stent überall dort geeignet sein, wo es gilt, einen Durchgang dauernd oder vorübergehend offen zu halten. Beispiele solcher Durchgänge sind der Gallengang und die Harnröhre.

Der Stent ist beispielsweise durch Extrusion in den unterschiedlichsten Längen sowie Aussen- und Innendurchmessern herstellbar. Somit kann in jedem Fall der Stent mit dem geeigneten Abmessungen zur Verfügung gestellt werden. Für die Behandlung einer Koronararterie wird beispielsweise ein Stent mit kleineren Aussen- und Innendurchmessern gewählt als für die Behandlung einer peripheren Arterie. Ebenfalls wird zur Behandlung eines bogenförmigen Gefässabschnittes in der Regel ein vergleichsweise kurzer Stent verwendet.

Nach einer Weiterbildung der Erfindung ist der Stent aus einem Werkstoff hergestellt, der in Körperflüssigkeit vollständig und biologisch abgebaut wird. Geeignet sind hier insbesondere Werkstoffe aus aliphatischen Polyestern und insbesondere aus Poly (_{E}-caprolacton). Der biologische Abbau dieser Kunststoffe ist bekannt. In der Medizin werden solche Stoffe bereits für die Fixation von Prothesen und als Kapseln für die kontrollierte Abgabe von Medikamenten verwendet. Die Abbaugeschwindigkeit ist beim Werkstoff des erfindungsgemässen Stent etwa so, dass er innerhalb von etwa 2 bis 6 Monaten vollständig oder zum grössten Teil aufgelöst ist. Da der Stent nach vergleichsweise kurzer Zeitdauer an der behandelten Stelle nicht mehr vorhanden ist, ist im Fall der Behandlung einer Stenose die Gefahr einer Trombose kleiner als bei einem dauernd verbleibenden Stent.

Ein Ausführungsbeispiel der Erfindung wird nachfolgend anhand der Zeichnungen näher erläutert. Es zeigen:
Fig. 1 eine perspektivische Ansicht eines erfindungsgemässen Stents,
Fig. 2a und 2b das vordere Ende eines Ballonkatheters mit einem erfindungsgemässen Stent vor und nach der Dilatation, und
Fig. 3a und 3b schematisch ein Schnitt durch ein Gefäss mit eingesetztem Stent vor und nach der Dilatation.

Der in Fig. 1 gezeigte Stent ist ein hohlzylindrischer Körper mit einer Länge C von beispielsweise 2 bis 10 cm. Für die Behandlung einer Koronararterie beträgt der Innendurchmesser B beispielsweise 1,0 ± 0,005 mm und der Aussendurchmesser A 1,6 mm. Zur Behandlung einer peripheren Arterie beträgt der Innendurchmesser B beispielsweise 2,0 mm und der Aussendurchmesser A 3,0 mm. Der Stent 1 kann durch Extrusion hergestellt werden. Als Werkstoff eignet sich ein Kunststoff, der im Temperaturbereich von 45 bis 75 Celsius schmilzt oder durch allmähliche Erweichung in den plastischen Zustand übergeht. Als Werkstoff eignen sich aliphatische Poyester und insbesondere Poly (_{E}-caprolacton). Geeignet sind auch Polymere, die unterhalb einer Temperatur von 45 _{°} Celsius fest sind und die wenigstens oberhalb einer Temperatur von etwa 70 Celsius in einen nicht kristallinen Zustand übergehen. Geeignete Polymere sind Polycaprolactone, Polyuretane sowie Polyamide.

Von diesen Polymeren sind diejenigen besonders geeignet, die in Körperflüssigkeit-biologisch abgebaut werden. Geeignet ist hier insbesondere Poly (_{E}-caprolacton), dessen Abbaubarkeit In Vivo im Journal of Applied Polymer Sciences, Vol. 26, 3779-3787 (1981) beschreiben ist.

Um den Stent 1 an die gewünschte zu behandelnde Stelle zu bringen, wird ein heizbarer Ballonkatheter 3 verwendet. Dieser besitzt einen dreilumigen Schaft 2, wobei ein Lumen 2a zur Durchführung eines Führungsdrahtes dient. In den beiden anderen Lumina 2b und 2c zirkuliert eine Salzlösung, die über hier nicht gezeigte Oeffnungen in den Innenraum des Ballons 3 ein und wieder ausströmt. Die Salzlösung wird mit einer am proximalen Ende des Schaftes 2 angeordneten Heiz- und Pumpeinrichtung erwärmt und gefördert. Es können hier jedoch auch Ballonkatheter verwendet werden, die in an sich bekannter Weise elektrisch, mit Hochfrequenz oder Mikrowellen geheizt werden.

Um den Stent 1 auf dem Ballonkatheter zu befestigen wird dieser vom distalen Ende her auf den gefalteten Ballon aufgeschoben. In vielen Fällen ist der Stent 1 durch die Reibung seiner Innenseite 1 an der Aussenseite 3a des Ballons 3 gegen eine Verschiebung in Längsrichtung genügent fixiert. In den übrigen Fällen wird der Druck im Ballon 3 leicht erhöht. Wie in Fig. 2a gezeigt, wird die Länge des Ballons 3 so gewählt, dass diese etwas grösser ist als die Länge C des Stent 1. Der Stent 1 liegt somit mit seiner ganzen Innenfläche 1 an der Aussenfläche 3a des Ballons an. Bei noch nicht geheiztem Ballonkatheter ist der Stent 1 vergleichsweise fest und steif und wird auch bei einem vergleichsweise hohem Druck im Ballon 3 nicht gedehnt.

Der Katheter wird mit dem aufgesetzten Stent 1 in an sich bekannter Weise eingeführt. Zur Behandlung einer Stenose wird der Katheter mit Hilfe eines hier nicht gezeigten Führungsdrahtes perkutan eingeführt. Die Position des Stent 1 kann mit Hilfe bekannter Markierungsstreifen 4 beispielsweise röntgenographisch verfolgt werden.

Ist der Stent 1 mit dem Ballonkatheter in die gewünschte Stelle gebracht, so wird der Ballon 3 geheizt und der Stent 1 auf eine Temperatur gebracht, in welcher er durch eine Dilatation des Ballones 3 plastisch gedehnt werden kann. Zur Behandlung einer koronaren Arterie wird beispielsweise der Innendurchmesser auf 2,7 und der Aussendurchmesser auf 3,0 mm erhöht. Im Fall einer peripheren Arterie beträgt der Innendurchmesser des dilatierten Stent beispielsweise 5,4 mm und der Aussendurchmesser 6,0 mm. Die Wandstärke des dilatierten Stent 1', ist wie aus den Fig. 2a und 2b ersichtlich wesentlich kleiner als beim ursprünglichen Stent 1.

Der Stent 1 wird soweit dilatiert, bis seine Aussenseite mit leichtem Druck an der Innenseite des Gefässes anliegt. Dies ist in den Fig. 3a und 3b schematisch gezeigt. Das Gefäss 5 ist in diesem Fall beispielsweise ein stenosierter Arterienabschnitt 5.

Ist der Stent 1' im Gefäss 5 fixiert, so wird die Heizung des Katheters unterbrochen, worauf die Temperatur des Stent 1' auf die Körpertemperatur sinkt und hierbei wieder den festen Zustand annimmt. Anschliessend wird der Druck im Ballon 3 vermindert und der Katheter in bekannter Weise aus dem Gefäss entfernt.

Ist der Stent 1' aus Poly (_{E}-caprolacton) hergestellt, so wird er in etwa 2 bis 6 Monaten autokatalytisch durch eine hydrolytische Esterspaltung vollständig abgebaut.

## Patentansprüche (Patentansprüche für folgende(n) Vertragsstaat(en) : AT, BE, CH, DE, FR, GB, IT, LI, LU, NL, SE)

1. Stent (1), insbesondere intravaskulärer Stent für Angioplastie, der ein hohlzylindrischer Körper ist und aufgeschoben auf den Ballon eines Ballonkatheters (3) perkutan einsetzbar und zu seiner Fixierung durch Dilatation des Ballons aufweitbar ist, dadurch gekennzeichnet, daß er aus einem Werkstoff hergestellt ist, der einen Schmelzpunkt oder einen Erweichungsbereich im Bereich von 45 bis 75 _{°} C aufweist, wobei der Stent eine Länge von 2 bis 10 cm aufweist.

2. Stent nach Patentanspruch 1, dadurch gekennzeichnet, dass der Werkstoff in Körperflüssigkeit biologisch abbaubar ist.

3. Stent nach Patentanspruch 1 oder 2, dadurch gekennzeichnet, dass der Werkstoff ein Polymer ist.

4. Stent nach einem der Patentansprüche 1 bis 3, dadurch gekennzeichnet, dass der Werkstoff ein aliphatischer Polyester ist.

5. Stent nach einem der Patentansprüche 1 bis 4, dadurch gekennzeichnet, dass der Werkstoff Polycaprolacton und insbesondere Poly (_{E}-caprolacton) ist.

6. Stent nach einem der Patentansprüche 1 bis 5, dadurch gekennzeichnet, dass er einen Innendurchmesser von 2 mm und einen Aussendurchmesser von 3 mm aufweist.

7. Ballonkatheter (3) zum Einsetzen eines Stent (1) gemäss einem der Patentansprüche 1 bis 6, der einen 3-lumigen Schaft aufweist, dadurch gekennzeichnet, dass er lediglich einen einzigen Ballon aufweist, dass er wenigstens im Bereich des Ballons heizbar ist, dass zwei der Lumen (2b,2c) derart mit dem Balloninnenraum verbunden sind, dass letzterer mit einer durchfliessenden Flüssigkeit heizbar ist und dass in das dritte Lumen (2a) ein Führungsdraht eingesetzt ist.

8. Ballonkatheter nach Anspruch 7, mit einem auf den Ballon (3) aufgesetzten Stent (1), dadurch gekennzeichnet, dass sich der Stent (1) im wesentlichen über die gesamte Länge des Ballons (3) erstreckt.

## Patentansprüche (Patentansprüche für folgende(n) Vertragsstaat(en) : : DK, ES, GR)

1. Stent, (1) insbesondere intravaskulärer Stent für Angioplastie, der aufgeschoben auf den Ballon eines Ballonkatheters (3) perkutan einsetzbar und zu seiner Fixierung durch Dilatation des Ballons aufweitbar ist, dadurch gekennzeichnet, dass er aus einem Werkstoff hergestellt ist, der einen Schmelzpunkt oder einen Erweichungsbereich im Bereich von 45 bis 75 _{°} Celsius aufweist.

2. Stent nach Patentanspruch 1, dadurch gekennzeichnet, dass der Werkstoff in Körperflüssigkeit biologisch abbaubar ist.

3. Stent nach Patentanspruch 1 oder 2, dadurch gekennzeichnet, dass der Werkstoff ein Polymer ist.

4. Stent nach einem der Patentansprüche 1 bis 3, dadurch gekennzeichnet, dass der Werkstoff ein aliphatischer Polyester ist.

5. Stent nach einem der Patentansprüche 1 bis 4, dadurch gekennzeichnet, dass der Werkstoff polycaprolacton und insbesondere poly (_{E}-caprolacton) ist.

6. Stent nach einem der Patentansprüche 1 bis 5, dadurch gekennzeichnet, dass er ein hohlzylindrischer Körper ist.

7. Ballonkatheter zum Einsetzen eines Stent gemäss einem der Patentansprüche 1 bis 6, dadurch gekennzeichnet, dass er wenigstens im Bereich des Ballons heizbar ist.

8. Katheter nach Patentanspruch 7, dadurch gekennzeichnet, dass der Schaft des Katheters wenigstens dreilumig ist, wobei zwei der Lumina derart mit dem Balloninnenraum verbunden sind, dass letzterer mit einer durchfliessenden Flüssigkeit heizbar ist.

## Claims (Claims for the following Contracting State(s) : AT, BE, CH, DE, FR, GB, IT, LI, LU, NL, SE)

1. A stent (1), more particularly an intravascular stent for angioplasty, which is a hollow cylindrical body and, when pushed onto the balloon of a balloon catheter (3), may be inserted percutaneously and widened for the purpose of fixing by dilating the balloon, characterised in that it is made of a material which has a melting point or a softening range in the range from 45 to 75 _{°} C, the stent having a length of 2 to 10 centimetres.

2. A stent according to claim 1, characterised in that the material is biodegradable in body fluid.

3. A stent according to claim 1 or 2, characterised in that the material is a polymer.

4. A stent according to one of claims 1 to 3, characterised in that the material is an aliphatic polyester.

5. A stent according to any one of claims 1 to 4, characterised in that the material is polycaprolactone, and more particularly poly (_{E}- caprolactone).

6. A stent according to any one of claims 1 to 5, characterised in that it has an inside diameter of 2 mm and an outside diameter of 3 mm.

7. A balloon catheter (3) for introducing a stent (1), according to any one of claims 1 to 6, which has a 3 lumen shaft, characterised in that it has only one balloon, but it is heatable at least in the region of the balloon, that two of the lumina (2b,2c) are connected to the inside of the balloon so that the latter may be heated by a fluid passing through it and that a guide wire is inserted into the third lumen (2a).

8. A balloon catheter according to claim 7, comprising a stent (1) pushed onto the balloon (3), characterised in that the stent (1) extends substantially over the entire length of the balloon (3).

## Claims (Claims for the following Contracting State(s) : DK, ES, GR)

1. A stent (1), more particularly an intravascular stent for angioplasty, which, when pushed onto the balloon of a balloon catheter (3), may be inserted percutaneously and widened for the purpose of fixing by dilating the balloon, characterised in that it is made of a material which has a melting point or a softening range in the range from 45 to 75 _{°} C.

2. A stent according to claim 1, characterised in that the material is biodegradable in body fluid.

3. A stent according to claim 1 or 2, characterised in that the material is a polymer.

4. A stent according to one of claims 1 to 3, characterised in that the material is an aliphatic polyester.

5. A stent according to any one of claims 1 to 4, characterised in that the material is polycaprolactone, and more particularly poly (_{E}- caprolactone).

6. A stent according to any one of claims 1 to 5, characterised in that it is a hollow cylindrical body.

7. A balloon catheter for introducing a stent according to any one of claims 1 to 6, characterised in that it is heatable at least in the region of the balloon.

8. A catheter according to claim 7, characterised in that the shaft of the catheter has at least three lumina, two of the lumina being connected to the inside of the balloon in such a way that the latter may be heated by a fluid passing through it.

## Revendications (Revendications pour l'(les) Etat(s) contractant(s) suivant(s) : suivants : AT, BE, CH, DE, FR, GB, IT, LI, LU, NL, SE)

1. Dilatateur (1), notamment dilatateur intravas- culaire pour angioplastie, qui est constitué par un corps creux et cylindrique enfoncé sur le ballon d'un cathéter à ballon (3) et pouvant être mis en place par voie cutanée et expansé en vue de sa fixation par dilatation du ballon, caractérisé en ce qu'il est réalisé en un matériau dont le point de fusion ou la plage de ramollissement est située entre 45 à 75 _{°} C, le dilatateur ayant une longueur de 2 à 10 cm.

2. Dilatateur selon la revendication 1, caractérisé en ce que le matériau est biologiquement décomposable dans le fluide corporel.

3. Dilatateur selon la revendication 1 ou 2, caractérisé en ce que le matériau est un polymère.

4. Dilatateur selon l'une des revendications 1 à 3, caractérisé en ce que le matériau est un polyester aliphatique.

5. Dilatateur selon l'une des revendications 1 à 4, caractérisé en ce que le matériau est de la polycaprolactone et notamment de la poly (_{E}- caprolactone).

6. Dilatateur selon l'une des revendications 1 à 5, caractérisé en ce que son diamètre interne est de 2 mm et son diamètre externe est de 3 mm.

7. Cathéter à ballon (3) pour la mise en place d'un dilatateur (1) selon l'une des revendications 1 à 6, qui comprend une tige à trois lumières, caractérisé en ce qu'il comprend simplement un unique ballon, en ce qu'il peut être chauffé au moins dans la région du ballon, en ce que deux des lumières (2b, 2c) sont reliées avec l'espace interne du ballon de manière que ce dernier puisse être chauffé par un fluide qui y passe et en ce qu'un fil de guidage est inséré dans la troisième lumière (2a).

8. Cathéter à ballon selon la revendication 7, comprenant un dilatateur (1) monté sur le ballon (3), caractérisé en ce que le dilatateur (1) s'étend sensiblement sur la totalité de la longueur du ballon (3).

## Revendications (Revendications pour l'(les) Etat(s) contractant(s) suivant(s) : suivants : DK, ES, GR)

1. Dilatateur (1), notamment dilatateur intravas- culaire pour angioplastie, qui, poussé sur le ballon d'un cathéter à ballon (3), peut être inséré par voie cutanée et être expansé en vue de sa fixation par dilatation du ballon, caractérisé en ce qu'il est réalisé en un matériau dont le point de fusion ou la plage de ramollissement est située entre 45 et 75 ° C.

2. Dilatateur selon la revendication 1, caractérisé en ce que le matériau peut être décomposé biologiquement dans le fluide corporel.

3. Dilatateur selon la revendication 1 ou 2, caractérisé en ce que le matériau est un polymère.

4. Dilatateur selon l'une des revendications 1 à 3, caractérisé en ce que le matériau est un polyester aliphatique.

5. Dilatateur selon l'une des revendications 1 à 4, caractérisé en ce que le matériau est de la polycaprolactone et en particulier de la poly (_{E}- caprolactone).

6. Dilatateur selon l'une des revendications 1 à 5, caractérisé en ce qu'il est constitué par un corps creux et cylindrique.

7. Cathéter à ballon destiné à la mise en place d'un dilatateur selon l'une des revendications 1 à 6, caractérisé en ce qu'il peut être chauffé au moins dans la région du ballon.

8. Cathéter selon la revendication 7, caractérisé en ce que la tige du cathéter comprend au moins trois lumières, deux des lumières étant reliées à l'espace interne du ballon de manière que ce dernier puisse être chauffé par un fluide qui y passe.
